# FASCICULE DE BREVET EUROPEEN

(11) **EP 2 400 879 B1**
(45) Date de publication et mention de la délivrance du brevet: **12.12.2012**
(21) Numéro de dépôt: 10710067.9
(22) Date de dépôt: 24.02.2010
(51) Int. Cl.: A61B 1/018

(54) **DISPOSITIF MULTIFONCTIONS D'EXPLORATION ET/OU D'INTERVENTION, NOTAMMENT À USAGE MÉDICAL.**
MULTIFUNKTIONSGERÄT FÜR UNTERSUCHUNGEN UND/ODER EINGRIFFE, INS BESONDERE FÜR MEDIZINISCHE ZWECKE
MULTIFUNCTIONAL DEVICE FOR EXPLORATION AND/OR INTERVENTION, ESPECIALLY FOR MEDICAL USE

(30) Priorité: 25.02.2009 FR 0900853
(43) Date de publication de la demande: 04.01.2012
(73) Titulaire: Pineau, Charles-Henri, 82000 Montauban (FR); Coupellier, Hervé, 82170 Fabas (FR)
(72) Inventeur: Pineau, Charles-Henri, 82000 Montauban (FR); Coupellier, Hervé, 82170 Fabas (FR)
(74) Mandataire: Morelle, Guy Georges Alain
(86) Numéro de dépôt international: PCT/FR2010/000173
(87) Numéro de publication internationale: WO 2010/097526

(56) Documents cités:
- EP-A- 1 665 993
- US-A- 5 323 768
- US-A- 5 741 271
- US-A1- 2005 085 692

## Description

L'invention concerne un dispositif multifonctions d'exploration et/ou d'intervention, notamment à usage médical.

Lors d'examens médicaux tels que par exemple des endoscopies, il est fréquent que le spécialiste soit amené à réaliser une intervention délicate nécessitant plusieurs instruments.

A l'heure actuelle, une solution consiste, à cet effet, à utiliser un endoscope dit à double canal opérateur. Toutefois, de tels endoscopes sont d'un prix de revient élevé et peu d'établissements sont en mesure de les mettre à disposition des praticiens.

De ce fait, la technique la plus couramment mise en oeuvre consiste à utiliser successivement différents instruments tels que notamment décrits dans la demande de brevet EP1 665 993, comprenant un corps de poignée creux prolongé d'un conduit souple, dit cathéter principal, et un chariot monté coulissant longitudinalement le long du corps de poignée, relié à un instrument par un lien mécanique s'étendant dans le cathéter principal de façon à permettre d'actionner et/ou de déplacer le dit instrument lors des déplacements longitudinaux du dit chariot.

Ainsi, à titre d'exemple, le geste de mucosectomie nécessite trois temps successifs nécessitant chacun un matériel distinct : coloration, décollement, section. Toutefois, une telle technique conduit, d'une part, à un surcoût financier, et d'autre part et surtout, à une perte de temps qui peut s'avérer préjudiciable du fait qu'elle intervient dans un moment très délicat.

La présente invention vise à pallier cet inconvénient et a pour principal objectif de fournir un dispositif multifonctions intégrant plusieurs instruments aptes à être positionnés ensemble au niveau d'un lieu d'intervention et pouvant être actionnés individuellement ou simultanément, éventuellement par un même opérateur.

Un autre objectif de l'invention est de fournir un dispositif multifonctions dont l'encombrement est comparable à celui d'un instrument classique.

A cet effet, l'invention vise un dispositif multifonctions d'exploration et/ou d'intervention, notamment à usage médical, comprenant :
- un corps de poignée creux prolongé d'un conduit souple, dit cathéter principal,
- un chariot, dit chariot d'extrémité, monté coulissant longitudinalement le long d'un tronçon d'extrémité du corps de poignée situé à l'opposé du cathéter principal, relié à un instrument par un lien mécanique s'étendant dans le dit cathéter principal de façon à permettre d'actionner et/ou de déplacer le dit instrument lors des déplacements longitudinaux du dit chariot d'extrémité.

Selon l'invention, ce dispositif multifonctions se caractérise en ce que :
- le corps de poignée comporte au moins un tronçon intermédiaire présentant une paroi périphérique dans laquelle est ménagée une fenêtre longitudinale,
- un chariot, dit chariot intermédiaire, est monté coulissant longitudinalement le long de chaque tronçon intermédiaire du corps de poignée, chacun des dits chariots intermédiaires comportant un appendice :
   ■ de forme adaptée pour se loger dans le corps de poignée au travers de la fenêtre longitudinale ménagée dans le tronçon intermédiaire correspondant,
   ■ percé d'un alésage longitudinal dans le prolongement duquel est solidarisé un conduit souple, dit cathéter secondaire, s'étendant dans le cathéter principal,
   ■ et délimitant un conduit de cheminement du lien mécanique reliant le chariot d'extrémité et son instrument associé, et éventuellement de chaque cathéter secondaire associé à un chariot intermédiaire monté sur un tronçon intermédiaire situé entre le tronçon d'extrémité et le tronçon intermédiaire concerné,
- un chariot annexe est monté coulissant longitudinalement le long de chaque chariot intermédiaire, chacun des dits chariots annexes étant relié à un instrument par un lien mécanique s'étendant dans l'alésage ménagé dans l'appendice du dit chariot intermédiaire et dans le cathéter secondaire associé à ce chariot intermédiaire, de façon à permettre d'actionner et/ou de déplacer le dit instrument lors des déplacements longitudinaux du dit chariot annexe.

(Il est à noter que, selon l'invention, on entend définir par corps de poignée un corps rigide manipulable manuellement et doté d'embout(s) ou raccord(s) rigide(s) permettant le raccordement d'un cathéter principal constitué d'un tube souple sur le dit corps de poignée.)

Selon l'invention, deux instruments au moins, respectivement reliés au chariot d'extrémité et à chaque chariot annexe, peuvent être positionnés au voisinage immédiat d'un lieu d'intervention, puis peuvent être utilisés successivement ou simultanément. De plus, de façon primordiale, tous les chariots, intermédiaire(s) et d'extrémité, et donc les cathéters associés, secondaire(s) et principal, peuvent être déplacés indépendamment les uns des autres, offrant une possibilité de déplacement relatif des instruments en cours d'intervention, et de nombreuses possibilités de positionnement relatif des dits instruments.

De plus, le dispositif multifonctions est conçu de façon que chaque cathéter secondaire s'étende dans le cathéter principal logeant le lien mécanique reliant le chariot d'extrémité à son instrument associé. Ainsi, la section du cathéter principal est comparable à celle du cathéter d'un instrument classique actuel.

Selon un mode de réalisation avantageux, le dispositif multifonctions selon l'invention comporte, en outre, un embout de raccordement d'un dispositif d'injection de fluide, ménagé dans un tronçon d'extrémité du corps de poignée opposé au tronçon d'extrémité sur lequel est monté le chariot d'extrémité, et adapté pour déboucher dans le corps de poignée et communiquer avec le cathéter principal.

Ainsi le cathéter principal est donc non seulement conçu pour loger le lien mécanique reliant le chariot d'extrémité à l'instrument associé, ainsi que chaque cathéter secondaire, mais forme également, sans augmentation de son encombrement, un conduit d'écoulement d'un fluide.

Une telle disposition confère une fonction supplémentaire d'injection, dans le cathéter principal, d'un fluide, tel que par exemple liquide de nettoyage, de coloration ..., au dispositif selon l'invention.

De plus, selon ce mode de réalisation, le cathéter principal loge avantageusement une membrane transversale positionnée au voisinage de l'extrémité libre du dit cathéter principal, la dite membrane étant percée d'orifices de passage de chaque cathéter secondaire et du lien mécanique reliant le chariot d'extrémité et son instrument associé, et étant percée d'orifices d'injection aptes à permettre une injection du fluide sous forme d'un spray.

De plus, dans la même optique de permettre une injection du fluide sous forme d'un spray, le cathéter principal comporte avantageusement un tronçon d'extrémité de forme évasée.

Selon un autre mode de réalisation avantageux de l'invention, le dispositif multifonctions selon l'invention comporte également un embout d'introduction d'un fil guide, ménagé dans un tronçon d'extrémité du corps de poignée opposé au tronçon d'extrémité sur lequel est monté le chariot d'extrémité, et adapté pour permettre l'introduction d'un fil guide dans le cathéter principal.

Ainsi le cathéter principal est donc non seulement conçu pour loger le lien mécanique reliant le chariot d'extrémité à l'instrument associé, ainsi que chaque cathéter secondaire, mais forme également, sans augmentation de son encombrement, un conduit d'introduction d'un fil guide.

Selon une autre caractéristique avantageuse de l'invention, chaque chariot intermédiaire comporte une coque de forme adaptée pour coiffer le corps de poignée.

De plus, chaque chariot intermédiaire et le corps de poignée présentent des sections comportant alors avantageusement au moins une arête longitudinale, adaptées pour obtenir un blocage relatif en rotation des dits chariot(s) intermédiaire(s) et corps de poignée.

Par ailleurs, en vue d'obtenir des positionnements relatifs stables de chaque chariot intermédiaire par rapport au corps de poignée, la coque de chaque chariot intermédiaire et le corps de poignée comportent avantageusement des faces en regard présentant des organes conjugués de blocage réversible en translation des dits éléments.

Selon une autre caractéristique avantageuse de l'invention, la coque de chaque chariot intermédiaire comporte une échancrure longitudinale définissant une glissière adaptée pour loger un chariot annexe formé d'un coulisseau apte à coulisser dans la dite glissière.

Par ailleurs, de façon avantageuse selon l'invention, au moins un chariot du dispositif multifonctions, chariot d'extrémité et/ou chariot annexe, est relié à son instrument associé par un lien mécanique électriquement conducteur, et comporte un raccord de branchement à un bistouri électrique, relié électriquement au lien mécanique associé au dit chariot.

D'autres caractéristiques, buts et avantages de l'invention ressortiront de la description détaillée qui suit en référence aux dessins annexés qui en représentent à titre d'exemple non limitatif un mode de réalisation préférentiel. Sur ces dessins :
- la figure 1 est une vue longitudinale de dessus d'un dispositif multifonctions selon l'invention,

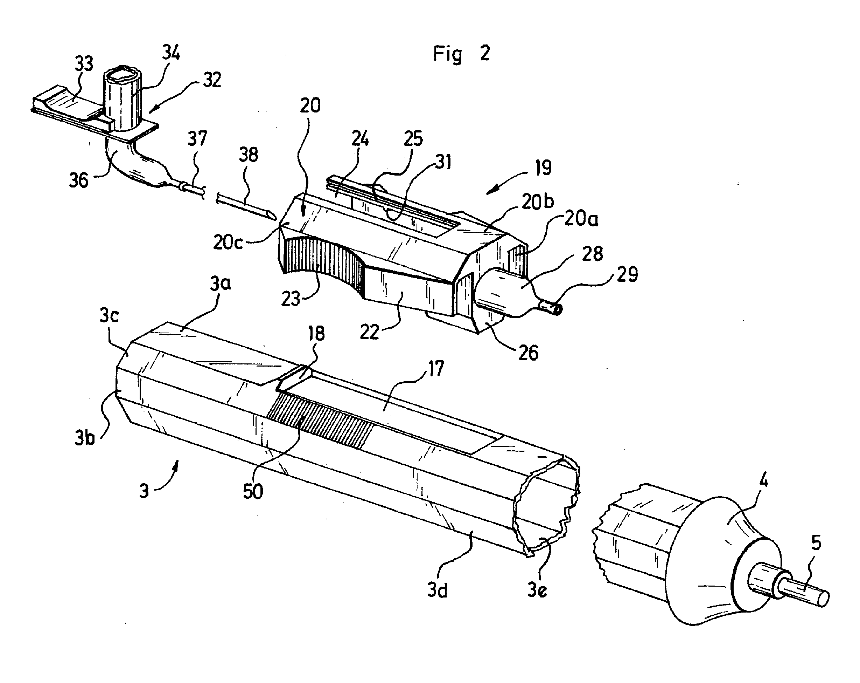

- la figure 2 est une vue en perspective éclatée représentant le tronçon intermédiaire d'un corps de poignée, un chariot intermédiaire et un chariot annexe d'un dispositif multifonctions selon l'invention,
- la figure 3a est une vue en perspective partielle représentant, assemblés, le tronçon intermédiaire d'un corps de poignée, un chariot intermédiaire et un chariot annexe d'un dispositif multifonctions selon l'invention,
- la figure 3b est une vue en perspective représentant, à une échelle agrandie, l'extrémité d'un cathéter principal et d'un cathéter secondaire ainsi que les instruments associés, d'un dispositif multifonctions selon l'invention,

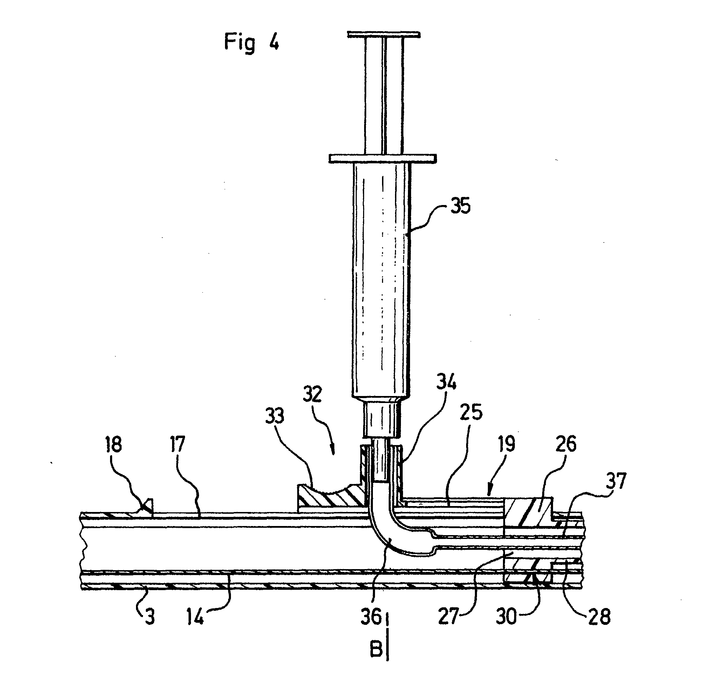

- la figure 4 est une coupe longitudinale partielle par un plan axial A d'un dispositif multifonctions selon l'invention dont le chariot annexe est équipé d'une seringue représentée non coupée,
- la figure 5 est une coupe transversale, par un plan B de la figure 4, du dispositif multifonctions selon l'invention,
- la figure 6 est une coupe transversale à échelle agrandie, par un plan C de la figure 7, du tronçon d'extrémité du cathéter principal d'un dispositif multifonctions selon l'invention,
- la figure 7 est une coupe longitudinale à échelle agrandie du tronçon d'extrémité du cathéter principal de ce dispositif multifonctions,
- et les figures 8a à 8d sont des schémas représentant quatre états de fonctionnement distincts du dispositif multifonctions selon l'invention.

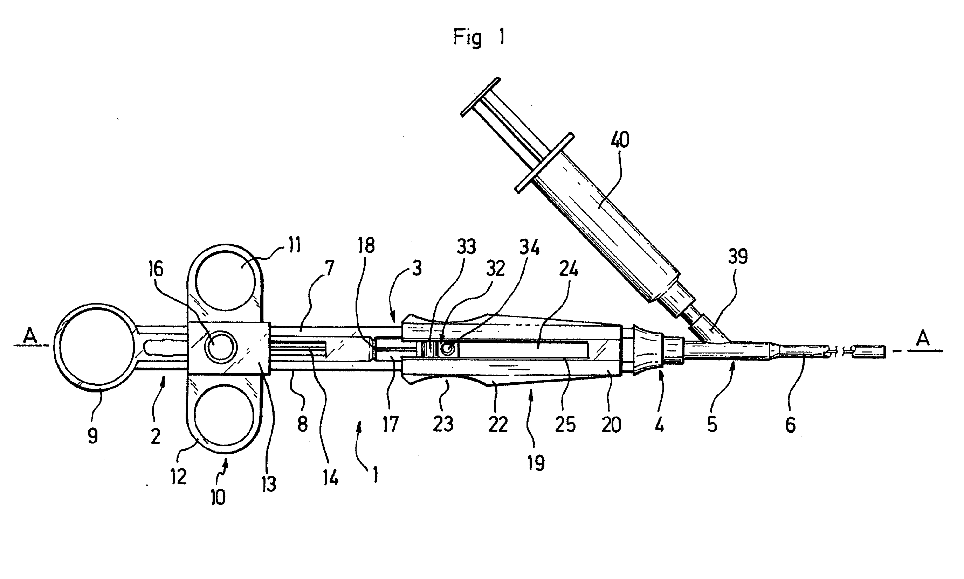

(A des fins de simplification et de clarté de la description, le dispositif multifonctions selon l'invention est décrit ci-dessous en considérant la figure 1 annexée comme une vue de dessus de ce dispositif, et les termes «supérieur, inférieur, latéral... », notamment, sont utilisés en conformité avec cette référence. De plus, les termes tels que postérieur et antérieur sont utilisés en considérant le dispositif tenu en main par un opérateur, les termes postérieur, arrière correspondant classiquement aux portions de ce dispositif les plus proches de la main de l'opérateur.)

Le dispositif multifonctions d'exploration et/ou d'intervention représenté à titre d'exemple aux figures est notamment destiné à un usage médical.

De plus, sur les figures annexées, ce dispositif multifonctions est équipé d'instruments particulièrement adaptés pour la pratique d'examens médicaux tels que des endoscopies.

Le dispositif multifonctions selon l'invention comporte, en premier lieu, un corps de poignée rigide 1 se subdivisant longitudinalement en :
- un tronçon d'extrémité postérieur 2,
- un tronçon intermédiaire 3,
- un raccord 4 de restriction de section de forme générale conique,
- et un conduit tubulaire antérieur 5 sur l'extrémité duquel est raccordé un tube souple 6 constituant le cathéter principal du dispositif multifonctions.

Le tronçon postérieur 2 de ce corps de poignée 1 se compose principalement de deux rails longitudinaux parallèles 7, 8 s'étendant à partir d'un anneau postérieur 9 de prise digitale, le long desquels est monté coulissant, sur une course « a » de l'ordre de huit centimètres, un chariot 10, dit chariot d'extrémité, composé :
- d'un noyau central 13 adapté pour coulisser le long des rails 7, 8,
- et de deux ailes latérales 11, 12 s'étendant de part et d'autre du noyau central 13, et constituées chacune d'un anneau de prise digitale.

Ce premier chariot 10 est relié à un instrument par un lien mécanique s'étendant dans le cathéter principal 6, et a pour fonction d'actionner et/ou de déplacer le dit instrument lors des déplacements longitudinaux du dit chariot.

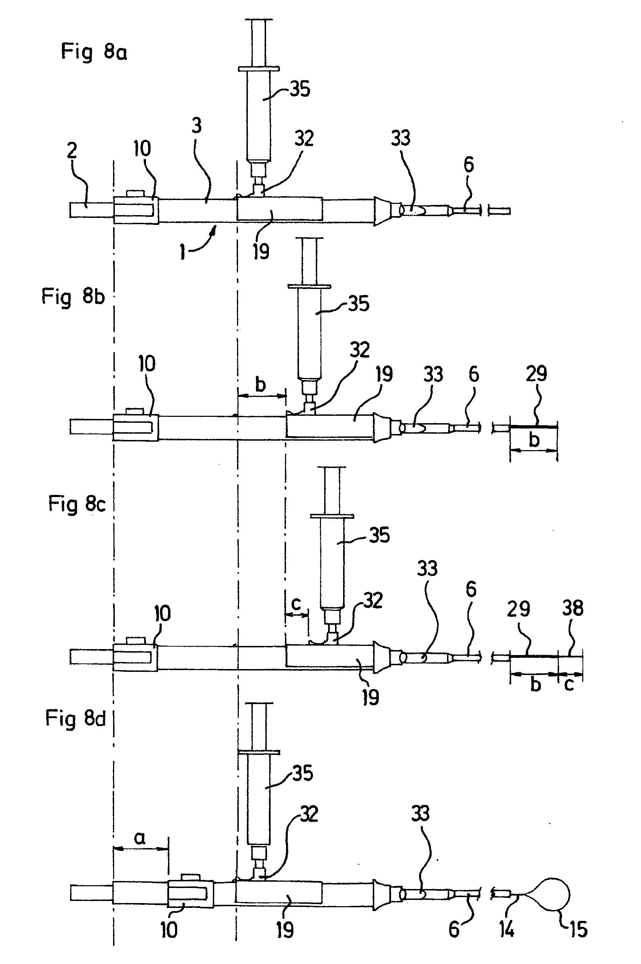

En l'exemple représenté aux figures, le lien mécanique consiste en un câble en un matériau électriquement conducteur 14 dont l'extrémité forme une boucle 15 constituant un instrument connu sous l'appellation de « anse » adapté pour :
- être logé à l'intérieur du cathéter principal 6, dans un état au moins partiellement fermé, dans la position reculée du chariot d'extrémité 10 représentée à la figure 8a,
- s'étendre dans le prolongement du cathéter principal 6, dans un état ouvert déployé, dans la position avancée du chariot d'extrémité 10 représentée à la figure 8d, où le dit chariot d'extrémité a subi une translation d'une course égale à « a » par rapport à sa position reculée.

Par ailleurs, le noyau central 13 du chariot d'extrémité 10 comporte un raccord 16 de branchement à un bistouri électrique, relié électriquement au câble 14 solidarisé au dit chariot, conférant une fonction supplémentaire d'instrument de coupe à l'anse 15.

Le tronçon intermédiaire 3 du corps de poignée 1 se compose, quant à lui, d'un corps tubulaire de section octogonale comportant une face supérieure 3a, deux faces latérales parallèles telles que 3b, deux faces obliques supérieures telles que 3c, de jonction de la face supérieure 3a avec les faces latérales 3b, deux faces obliques inférieures 3d et une face inférieure 3e.

En premier lieu concernant ce tronçon intermédiaire 3, la face supérieure 3a est percée d'une fenêtre longitudinale 17 de forme rectangulaire, présentant une longueur de l'ordre de huit centimètres. De plus, cette fenêtre est délimitée postérieurement par une ailette transversale de butée 18 ménagée de façon à s'étendre en saillie par rapport la face supérieure 3a.

Le dispositif multifonctions selon l'invention comporte, monté coulissant le long de ce tronçon intermédiaire 3, un chariot, dit chariot intermédiaire, 19 composé principalement :
- d'une coque 20 de forme générale semi octogonale adaptée pour coiffer la face supérieure 3a, les faces supérieures obliques 3c et les faces latérales 3b du tronçon intermédiaire 3,
- et d'un appendice 26 positionné au niveau de l'extrémité antérieure du chariot intermédiaire 19, et de forme générale parallélépipédique rectangle adaptée pour s'étendre à l'intérieur du tronçon intermédiaire 3 du corps de poignée 1 au travers de la fenêtre 17.

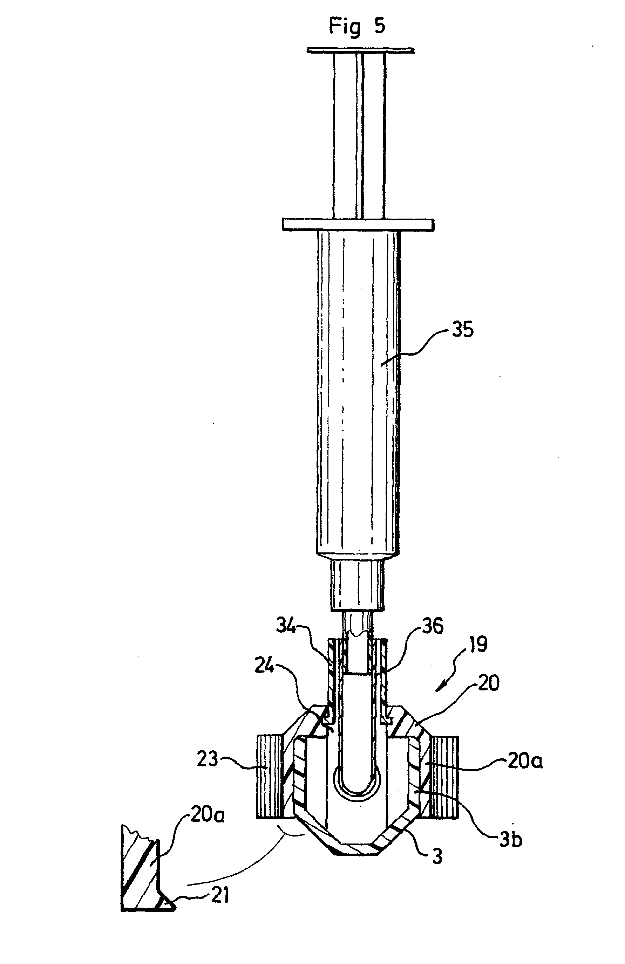

La coque 20 de ce chariot intermédiaire 19 comporte, en premier lieu, deux parois latérales telles que 20a agencées pour s'étendre en recouvrement des faces latérales 3b du tronçon intermédiaire 3, et prolongées chacune d'un rebord longitudinal oblique 21, représenté à la figure 5, adapté pour former un crochet élastique d'encliquetage du chariot intermédiaire 19 sur le tronçon intermédiaire 3.

Chacune de ces parois latérales 20a comporte, en outre, une surépaisseur 22 formée sur la face extérieure de la coque 20, et dans laquelle est creusée une empreinte longitudinale postérieure 23 dotée d'une surface striée, de prise digitale du chariot intermédiaire 19.

La coque 20 du chariot intermédiaire 19 comporte, en outre, une face supérieure 20b dans laquelle est ménagée une échancrure 24 s'étendant à partir de l'extrémité postérieure de la dite face supérieure, dont les bords intérieurs longitudinaux 25 sont conformés de façon à définir une glissière adaptée pour loger un chariot annexe 32 consistant en un coulisseau.

La coque 20 comporte, enfin, deux faces supérieures obliques 20c en sous-face de chacune desquelles est ménagé un cran transversal 31 de blocage réversible adapté pour coopérer avec des stries transversales 50 ménagées sur les faces supérieures obliques 3c du tronçon intermédiaire 3, de façon à obtenir des positionnements relatifs stables du chariot intermédiaire 19 le long du corps de poignée 1.

L'appendice 26 du chariot intermédiaire 19 est quant à lui, en premier lieu, percé d'un alésage longitudinal 27, et comporte, dans le prolongement du dit alésage, un embout 28 de raccordement d'un tube souple 29 constituant un cathéter dit secondaire adapté pour s'étendre dans le cathéter principal 6. De plus, cet appendice 26 est également percé d'un conduit 30 de cheminement du câble 14 reliant le chariot d'extrémité 10 et son instrument (anse) 15 associé.

Les dimensions respectives de la fenêtre 17 et de l'appendice 26 du chariot intermédiaire 19 sont adaptées pour permettre un déplacement longitudinal du dit chariot intermédiaire sur une course « b » de l'ordre de quatre centimètres, entre :
- une position reculée du chariot intermédiaire 19, représentée à la figure 8a, dans laquelle le dit chariot intermédiaire est en butée contre l'ailette de butée 18, et le cathéter secondaire 29 est escamoté à l'intérieur du cathéter principal 6,
- et une position avancée du chariot intermédiaire 19, représentée à la figure 8b, dans laquelle un tronçon d'extrémité d'une longueur sensiblement égale à « b » du cathéter secondaire 29, s'étend dans le prolongement de l'extrémité du cathéter principal 6.

De plus, lors de cette course, des positions intermédiaires stables sont définies par la coopération des crans de blocage 31 avec les stries 50.

Il est à noter que le chariot d'extrémité 10 du dispositif multifonctions selon l'invention peut également être constitué d'un chariot de même conception que le chariot intermédiaire ci-dessus décrit.

Tel que cité ci-dessus, le dispositif multifonctions selon l'invention comporte également un troisième chariot 32, dit chariot annexe, constitué d'un coulisseau apte à se déplacer longitudinalement par rapport au chariot intermédiaire 19.

Ce coulisseau 32 comporte une face supérieure sur laquelle est ménagée une empreinte longitudinale postérieure 33 dotée d'une surface striée, d'actionnement digital du dit coulisseau.

De plus, ce coulisseau 32 comporte un embout 34 de raccordement d'une seringue 35 communiquant, par l'intermédiaire d'un raccord coudé 36, avec un conduit souple 37 adapté pour s'étendre dans l'alésage 27 ménagé dans l'appendice 26 du chariot intermédiaire 19 puis dans le cathéter secondaire 29, et sur l'extrémité duquel est montée une aiguille d'injection 38.

Les dimensions respectives de l'échancrure 24 et du coulisseau 32 sont adaptées pour permettre un déplacement longitudinal du chariot annexe sur une course utile « c » de l'ordre de 1,5 centimètres, entre :
- une position avancée du chariot annexe 32, représentée à la figure 8c, dans laquelle un tronçon d'injection d'une longueur sensiblement égale à « c » de l'aiguille 38 s'étend dans le prolongement de l'extrémité du cathéter secondaire 29,
- et des positions reculées du chariot annexe 32, telle que celle représentée à la figure 8b, dans lesquelles l'aiguille 38 est escamotée à l'intérieur du cathéter secondaire 29. Il est à noter que la position extrême reculée du chariot annexe 32 correspond à une position de butée du dit chariot annexe contre l'ailette de butée 18.

De plus, en fonction des besoins liés aux instruments associés, le déplacement du chariot annexe peut consister soit en un déplacement unidirectionnel, soit en un déplacement bidirectionnel de part et d'autre d'une position neutre médiane.

Le dispositif multifonctions selon l'invention comporte, par ailleurs, un embout 39 de raccordement d'une seringue 40, disposé en position intermédiaire du conduit tubulaire antérieur 5, de façon à être en communication directe avec le cathéter principal 6. Tel que représenté à la figure 1, cet embout 39 s'étend dans un plan orthogonal au plan dans lequel s'étend l'embout 34, selon un axe incliné de 45° vers l'arrière par rapport à l'axe longitudinal du corps de poignée 1.

Une telle disposition permet d'injecter un fluide dans le cathéter principal 6, tel que par exemple un liquide de nettoyage, de coloration...

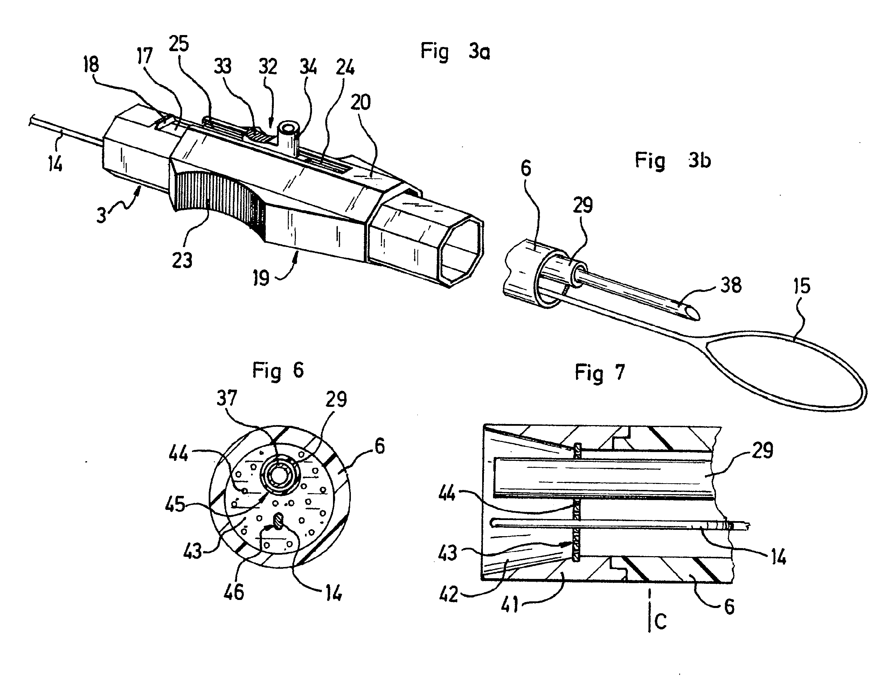

De plus, le cathéter principal 6 comporte alors, tel que représenté aux figures 6 et 7, un embout antérieur 41 adapté pour être monté, par exemple par emboîtement, sur l'extrémité libre du dit cathéter principal, le dit embout :
- comportant un tronçon d'extrémité 42 de forme interne évasée,
- logeant une membrane transversale 43 percée, d'une part, d'orifices 45, 46 de passage de chaque cathéter secondaire 29 et du câble 14 reliant le chariot d'extrémité 10 et son instrument 15 associé, et d'autre part, d'orifices d'injection 44 aptes à permettre une injection du fluide sous forme d'un spray.

Il est à noter que l'embout 39 peut être utilisé à des fins différentes et notamment consister en un embout d'introduction d'un fil guide adapté pour permettre l'introduction d'un fil guide dans le cathéter principal 6.

Dans ce cas, en outre, le cathéter principal 6 est dépourvu d'embout antérieur 41 et de membrane 43.

Le dispositif multifonctions décrit ci-dessus, doté d'une anse diathermique 15 reliée à un bistouri électrique, d'une aiguille à sclérose 38 et d'un canal 39 d'injection d'un liquide de coloration, est particulièrement destiné à être utilisé à des fins médicales en vue par exemple de pratiquer une mucosectomie.

En effet, le dispositif permet de mettre en oeuvre les trois étapes successives d'une mucosectomie, à savoir : coloration vitale, injection sous muqueuse et résection du polype.

En premier lieu, la coloration vitale est réalisée au moyen d'une seringue 40 connectée sur l'embout 39 permettant de délivrer le colorant directement dans le cathéter principal 6, puis de diffuser ce colorant sous forme d'un spray grâce à la membrane 43.

Lors de l'injection sous muqueuse, cette dernière est grandement facilitée par la présence, au niveau du lieu d'injection, du cathéter principal 6 et éventuellement de l'anse 15 qui permettent d'obtenir une meilleure exposition de la lésion, et une injection sous muqueuse de qualité.

De même, la troisième étape de résection du polype, effectuée au moyen de l'anse 15, est grandement facilitée par la présence du cathéter secondaire 29 et éventuellement de l'aiguille 38, qui permettent d'obtenir une meilleure exposition de la lésion à traiter.

Toutes ces opérations peuvent en outre être réalisées par un seul opérateur.

Un grand nombre d'autres applications, notamment dans le domaine médical, peuvent être envisagées en utilisant des dispositifs multifonctions selon l'invention dotés d'instruments spécifiques divers.

Ainsi, à titre d'exemples, il peut être cité les applications suivantes en endoscopie digestive :
- lavage/coloration - anse - pince,
- lavage/coloration - anse - « endoloop »,
- lavage/coloration - anse - clip hémostatique,
- lavage/coloration - couteau dissection sous muqueuse
   (« flush knife ») - pince.

Il peut également être cité comme autre application : sphinctéromie/infundibulotomie/fil guide...

## Revendications

1. Dispositif multifonctions d'exploration et/ou d'intervention, notamment à usage médical, comprenant :
- un corps de poignée creux (1) prolongé d'un conduit souple (6), dit cathéter principal,
- un chariot (10), dit chariot d'extrémité, monté coulissant longitudinalement le long d'un tronçon d'extrémité (2) du corps de poignée (1) situé à l'opposé du cathéter principal (1), relié à un instrument (15) par un lien mécanique (14) s'étendant dans le dit cathéter principal de façon à permettre d'actionner et/ou de déplacer le dit instrument lors des déplacements longitudinaux du dit chariot d'extrémité,
le dit dispositif multifonctions étant **caractérisé en ce que** :
- le corps de poignée (1) comporte au moins un tronçon intermédiaire (3) présentant une paroi périphérique (3a-3e) dans laquelle est ménagée une fenêtre longitudinale (17),
- un chariot (19), dit chariot intermédiaire, est monté coulissant longitudinalement le long de chaque tronçon intermédiaire (3) du corps de poignée (1), chacun des dits chariots intermédiaires comportant un appendice (26) :
■ de forme adaptée pour se loger dans le corps de poignée (1) au travers de la fenêtre longitudinale (17) ménagée dans le tronçon intermédiaire (3) correspondant,
■ percé d'un alésage longitudinal (27) dans le prolongement duquel est solidarisé un conduit souple (29), dit cathéter secondaire, s'étendant dans le cathéter principal (6),
■ et délimitant un conduit (30) de cheminement du lien mécanique (14) reliant le chariot d'extrémité (10) et son instrument (15) associé, et éventuellement de chaque cathéter secondaire associé à un chariot intermédiaire monté sur un tronçon intermédiaire situé entre le tronçon d'extrémité et le tronçon intermédiaire concerné,
- un chariot annexe (32) est monté coulissant longitudinalement le long de chaque chariot intermédiaire (19), chacun des dits chariots annexes étant relié à un instrument (38) par un lien mécanique (37) s'étendant dans l'alésage (27) ménagé dans l'appendice (26) du dit chariot intermédiaire et dans le cathéter secondaire (29) associé à ce chariot intermédiaire, de façon à permettre d'actionner et/ou de déplacer le dit instrument lors des déplacements longitudinaux du dit chariot annexe.

2. Dispositif multifonctions selon la revendication 1 **caractérisé en ce qu'**il comporte un embout (39) de raccordement d'un dispositif d'injection de fluide (40), ménagé dans un tronçon d'extrémité (5) du corps de poignée (1) opposé au tronçon d'extrémité (2) sur lequel est monté le chariot d'extrémité (10), et adapté pour déboucher dans le corps de poignée (1) et communiquer avec le cathéter principal (6).

3. Dispositif multifonctions selon la revendication 2 **caractérisé en ce que** le cathéter principal (6) loge une membrane transversale (43) positionnée au voisinage de l'extrémité libre (41) du dit cathéter principal, la dite membrane étant percée d'orifices (45, 46) de passage de chaque cathéter secondaire (29) et du lien mécanique (14) reliant le chariot d'extrémité (10) et son instrument (15) associé, et étant percée d'orifices d'injection (44) aptes à permettre une injection du fluide sous forme d'un spray.

4. Dispositif multifonctions selon l'une des revendications 2 ou 3 **caractérisé en ce que** le cathéter principal (6) comporte un tronçon d'extrémité (42) de forme évasée.

5. Dispositif multifonctions selon l'une des revendications 1 ou 2 **caractérisé en ce qu'**il comporte un embout (39) d'introduction d'un fil guide, ménagé dans un tronçon d'extrémité (5) du corps de poignée (1) opposé au tronçon d'extrémité (2) sur lequel est monté le chariot d'extrémité (10), et adapté pour permettre l'introduction d'un fil guide dans le cathéter principal (6).

6. Dispositif multifonctions selon l'une des revendications précédentes **caractérisé en ce que** chaque chariot intermédiaire (19) comporte une coque (20) de forme adaptée pour coiffer le corps de poignée (1).

7. Dispositif multifonctions selon la revendication 6 **caractérisé en ce que** chaque chariot intermédiaire (19) et le corps de poignée (1) présentent des sections comportant au moins une arête longitudinale, adaptées pour obtenir un blocage relatif en rotation des dits chariot(s) intermédiaire(s) et corps de poignée.

8. Dispositif multifonctions selon l'une des revendications 6 ou 7 **caractérisé en ce que** la coque (20) de chaque chariot intermédiaire (19) et le corps de poignée (1) comportent des faces en regard (3c, 20c) présentant des organes (31, 50) conjugués de blocage réversible en translation des dits éléments.

9. Dispositif multifonctions selon l'une des revendications 6 à 8 **caractérisé en ce que** la coque (20) de chaque chariot intermédiaire (19) comporte une échancrure longitudinale (24) définissant une glissière (25) adaptée pour loger un chariot annexe (32) formé d'un coulisseau apte à coulisser dans la dite glissière.

10. Dispositif multifonctions selon l'une des revendications précédentes **caractérisé en ce qu'**au moins un chariot, chariot d'extrémité (10) et/ou chariot annexe (32), est relié à son instrument (15, 38) associé par un lien mécanique (14, 37) électriquement conducteur, et comporte un raccord (16) de branchement à un bistouri électrique, relié électriquement au lien mécanique (14, 37) associé au dit chariot.

## Claims

1. Multifunctional device for exploration and/or intervention, especially for medical use, including:
- a hollow handle (1) extended by a flexible conduit (6), known as the main catheter,
- a carriage (10), known as the end carriage, mounted so as to slide longitudinally along an end portion (2) of the handle (1) located opposite the main catheter (6), connected to an instrument (15) by a mechanical connection (14) extending inside the said main catheter to allow the said instrument to be actuated and/or moved during longitudinal movements of the said end carriage,
The said multifunctional device being **characterised in that**:
- the handle (1) includes at least one intermediate portion (3) having a peripheral wall (3a-3e) in which is formed a longitudinal slot (17),
- a carriage (19), known as the intermediate carriage, is mounted so as to slide longitudinally along each intermediate portion (3) of the handle (1), each of the said intermediate carriages including an attachment (26):
• suitably shaped to fit into the handle (1) through the longitudinal slot (17) formed in the corresponding intermediate portion (3),
• pierced with a longitudinal slot (27) in the continuation of which is secured a flexible conduit (29), known as the secondary catheter, extending into the main catheter (6),
• and defining a conduit (30) for passage of the mechanical connection (14) linking the end carriage (10) and its associated instrument (15), and optionally of each secondary catheter associated with an intermediate carriage mounted on an intermediate portion located between the end portion and the intermediate portion concerned,
- an auxiliary carriage (32) is mounted so as to slide longitudinally along each intermediate carriage (19), each of the said auxiliary carriages being connected to an instrument (38) by a mechanical connection (37) extending into the slot (27) formed in the attachment (26) of the said intermediate carriage and in the secondary catheter (29) associated with this intermediate carriage, so as to enable the said instrument to be actuated and/or moved during longitudinal movements of the said auxiliary carriage.

2. Multifunctional device according to claim 1 **characterised in that** it includes an adapter (39) for connecting a fluid injection device (40), formed in an end portion (5) of the handle (1) opposite the end portion (2) whereon is mounted the end carriage (10), and adapted to emerge into the handle (1) and to communicate with the main catheter (6).

3. Multifunctional device according to claim 2 **characterised in that** the main catheter (6) houses a transverse membrane (43) positioned in the vicinity of the free end (41) of the said main catheter, the said membrane being pierced with holes (45, 46) for the passage of each secondary catheter (29) and the mechanical connection (14) connecting the end carriage (10) and its associated instrument (15), and being pierced with injection holes (44) designed to allow an injection of fluid in the form of a spray.

4. Multifunctional device according to any one of claims 2 or 3 **characterised in that** the main catheter (6) includes an end portion (42) of tapered shape.

5. Multifunctional device according to any one of claims 1 or 2 **characterised in that** it includes an adapter (39) for introducing a guide wire, formed in an end portion (5) of the handle (1) opposite the end portion (2) whereon is mounted the end carriage (10), and adapted to allow the introduction of a guide wire into the main catheter (6).

6. Multifunctional device according to any one of the preceding claims **characterised in that** each intermediate carriage (19) includes a shell (20) suitably shaped to cover the handle (1).

7. Multifunctional device according to claim 6 **characterised in that** each intermediate carriage (19) and the handle (1) have sections including at least one longitudinal ridge adapted so as to obtain a relative locking in rotation of the said intermediate carriage(s) and the handle.

8. Multifunctional device according any one of claims 6 or 7 **characterised in that** the shell (20) of each intermediate carriage (19) and the handle (1) include opposing faces (3c, 20c) having conjugate members (31, 50) for reversibly locking the translational movement of the said elements.

9. Multifunctional device according to any one of claims 6 to 8 **characterised in that** the shell (20) of each intermediate carriage (19) includes a longitudinal notch (24) defining a runner (25) adapted to accommodate an auxiliary carriage (32) formed by a slide capable of sliding in said runner.

10. Multifunctional device according to any one of the preceding claims **characterised in that** at least one carriage, an end carriage (10) and/or auxiliary carriage (32), is connected to its associated instrument (15, 38) by an electrically conductive mechanical connection (14, 37), and includes a connector (16) for connecting to an electric scalpel, electrically connected to the mechanical connection (14, 37) associated with the said carriage.

## Patentansprüche

1. Multifunktionsvorrichtung für Untersuchungen und/oder Eingriffe, insbesondere für medizinische Zwecke, enthaltend:
- einen hohl ausgeführten Greifkörper (1), der sich über eine nachgiebige Leitung (6), dem sogenannten Hauptkatheter, fortsetzt,
- einen Schlitten (10), den sogenannten Endschlitten, der entlang eines dem Hauptkatheter (1) entgegengesetzten Endabschnitts (2) des Greifkörpers (1) longitudinal gleitbeweglich gelagert ist und über ein mechanisches Verbindungsmittel (14) mit einem Instrument (15) verbunden ist, das sich im Hauptkatheter erstreckt, so dass es möglich ist, das Instrument bei Längsverschiebungen des Endschlittens zu betätigen und/oder zu verlagern,
wobei die Multifunktionsvorrichtung **dadurch gekennzeichnet ist, dass**
- der Greifkörper (1) zumindest einen Zwischenabschnitt (3) aufweist, der eine Umfangswand (3a-3e) enthält, in welche ein longitudinal verlaufendes Fenster (17) eingebracht ist,
- ein Schlitten (19), der sogenannte Zwischenschlitten, entlang eines jeden Zwischenabschnitts (3) des Greifkörpers (1) longitudinal verschiebbar gelagert ist, wobei jeder der Zwischenschlitten einen Ansatz (26) aufweist, der
- in seiner Form dazu ausgelegt ist, durch das in den entsprechenden Zwischenabschnitt (3) eingebrachte longitudinale Fenster (17) hindurch in dem Greifkörper (1) aufgenommen zu werden,
- mit einer Längsbohrung (27) versehen ist, in deren Verlängerung eine nachgiebige Leitung (29), Nebenkatheter genannt, fest angebracht ist und die sich in dem Hauptkatheter (6) erstreckt,
- und eine Leitung (30) zum Führen des mechanischen Verbindungsmittels (14) begrenzt, welches den Endschlitten (10) und das diesem zugeordnete Instrument (15) verbindet, und gegebenenfalls zum Führen von jedem Nebenkatheter, der einem Zwischenschlitten zugeordnet ist, der an einem zwischen Endabschnitt und betreffendem Zwischenabschnitt liegenden Zwischenabschnitt gelagert ist,
- ein Zusatzschlitten (32) entlang eines jeden Zwischenschlittens (19) longitudinal verschiebbar gelagert ist, wobei jeder der Zusatzschlitten mit einem Instrument (38) über ein mechanisches Verbindungsmittel (37) verbunden ist, welches sich in der in den Ansatz (26) des Zwischenschlittens eingebrachten Bohrung (27) und in dem diesem Zwischenschlitten zugeordneten Nebenkatheter (29) erstreckt, so dass es möglich ist, das Instrument bei Längsverschiebungen des Zusatzschlittens zu betätigen und/oder zu verlagern.

2. Multifunktionsvorrichtung nach Anspruch 1, **dadurch gekennzeichnet, dass** sie ein Anschlussstück (39) zum Anschließen einer Fluidinjektionsvorrichtung (40) aufweist, das in einen Endabschnitt (5) des Greifkörpers (1) eingebracht ist, der dem Endabschnitt (2) entgegengesetzt ist, an dem der Endschlitten (10) gelagert ist, und dazu ausgelegt ist, in den Greifkörper (1) auszumünden und mit dem Hauptkatheter (6) zu kommunizieren.

3. Multifunktionsvorrichtung nach Anspruch 2, **dadurch gekennzeichnet, dass** der Hauptkatheter (6) eine quer verlaufende Membran (43) aufnimmt, die benachbart zum freien Ende (41) des Hauptkatheters angeordnet ist, wobei die Membran mit Durchtrittsöffnungen (45, 46) zum Durchtritt eines jeden Nebenkatheters (29) und des mechanischen Verbindungsmittels (14) versehen ist, welches den Endschlitten (10) und das diesem zugeordnete Instrument (15) verbindet, sowie mit Injektionsöffnungen (44) versehen ist, die dazu ausgelegt sind, eine Injektion des Fluids in Form eines Sprays zu ermöglichen.

4. Multifunktionsvorrichtung nach einem der Ansprüche 2 oder 3, **dadurch gekennzeichnet, dass** der Hauptkatheter (6) einen Endabschnitt (42) mit sich erweiternder Form aufweist.

5. Multifunktionsvorrichtung nach einem der Ansprüche 1 oder 2, **dadurch gekennzeichnet, dass** sie ein Einführstück (39) zum Einführen eines Führungsdrahtes aufweist, das in einen Endabschnitt (5) des Greifkörpers (1) eingebracht ist, welcher dem Endabschnitt (2) entgegengesetzt ist, an welchem der Endschlitten (10) gelagert ist, und das dazu ausgelegt ist, das Einführen eines Führungsdrahtes in den Hauptkatheter (6) zu ermöglichen.

6. Multifunktionsvorrichtung nach einem der vorangehenden Ansprüche, **dadurch gekennzeichnet, dass** jeder Zwischenschlitten (19) eine Schale (20) aufweist, die in ihrer Form dazu ausgelegt ist, den Greifkörper (1) zu überdecken.

7. Multifunktionsvorrichtung nach Anspruch 6, **dadurch gekennzeichnet, dass** jeder Zwischenschlitten (19) und der Greifkörper (1) Bereiche aufweisen, die zumindest eine Längskante enthalten und dazu ausgelegt sind, ein relative Drehsicherung für die Zwischenschlitten und den Greifkörper zu bewirken.

8. Multifunktionsvorrichtung nach einem der Ansprüche 6 oder 7, **dadurch gekennzeichnet, dass** die Schale (20) eines jeden Zwischenschlittens (19) und der Greifkörper (1) gegenüberliegende Seiten (3c, 20c) aufweisen, die zusammenhängende Glieder (31, 50) zur umkehrbaren Verschiebesicherung der genannten Teile enthalten.

9. Multifunktionsvorrichtung nach einem der Ansprüche 6 bis 8, **dadurch gekennzeichnet, dass** die Schale (20) eines jeden Zwischenschlittens (19) eine Längsaussparung (24) aufweist, die eine Gleitführung (25) definiert, welche dazu ausgelegt ist, einen Zusatzschlitten (32) aufzunehmen, der aus einem in der Gleitführung gleitbeweglichen Schieber gebildet ist.

10. Multifunktionsvorrichtung nach einem der vorangehenden Ansprüche, **dadurch gekennzeichnet, dass** zumindest ein Schlitten, der Endschlitten (10) und/oder der Zusatzschlitten (32), mit dem diesem zugeordneten Instrument (15, 38) über ein elektrisch leitfähiges, mechanisches Verbindungsmittel (14, 37) verbunden ist und einen Anschluss (16) zum Verbinden mit einem elektrischen Skalpell aufweist, der elektrisch mit dem dem Schlitten zugeordneten mechanischen Verbindungsmittel (14, 37) verbunden ist.
